# EUROPEAN PATENT SPECIFICATION

(11) **EP 3 244 885 B1**
(45) Date of publication and mention of the grant of the patent: **13.03.2019**
(21) Application number: 16707949.0
(22) Date of filing: 13.01.2016
(51) Int. Cl.: A61K 31/385, A61P 15/06

(54) **LIPOIC ACID FOR TREATING OR PREVENTING THREATENED MISCARRIAGE OR PRETERM DELIVERY**
LIPONSÄURE ZUR BEHANDLUNG ODER PRÄVENTION EINER DROHENDEN MISSGEBURT ODER EINER FRÜHGEBURT
ACIDE LIPOÏQUE POUR LE TRAITEMENT OU LA PRÉVENTION DU RISQUE D'ACCOUCHEMENT AVANT TERME OU D'AVORTEMENT SPONTANÉ

(30) Priority: 13.01.2015 IT FI20150004
(43) Date of publication of application: 22.11.2017
(73) Proprietor: LO.LI. PHARMA S.R.L., 00156 Roma (IT)
(72) Inventor: UNFER, Vittorio, 00155 Roma (IT)
(74) Representative: Capasso, Olga
(86) International application number: PCT/IB2016/050139
(87) International publication number: WO 2016/113679

(56) References cited:
- WO-A1-03/047567
- WO-A2-2007/138022
- UA-U- 17 059
- PADMANABHAN RENGASAMY ET AL: "Beneficial effect of supplemental lipoic acid on diabetes-induced pregnancy loss in the mouse.", ANNALS OF THE NEW YORK ACADEMY OF SCIENCES NOV 2006, vol. 1084, November 2006 (2006-11), pages 118-131, XP002743756, ISSN: 0077-8923
- MOORE ROBERT M ET AL: "Alpha-lipoic acid inhibits tumor necrosis factor-induced remodeling and weakening of human fetal membranes.", BIOLOGY OF REPRODUCTION APR 2009, vol. 80, no. 4, April 2009 (2009-04), pages 781-787, XP002743757, ISSN: 0006-3363
- S.L. MILLER ET AL: "Antioxidant Therapies: A Potential Role in Perinatal Medicine", NEUROENDOCRINOLOGY, vol. 96, no. 1, 1 January 2012 (2012-01-01), pages 13-23, XP055139010, ISSN: 0028-3835, DOI: 10.1159/000336378

## Description

### FIELD OF THE INVENTION

The present invention relates to the field of substances for pharmaceutical use, in particular it relates to a new medical use of lipoic acid.

### BACKGROUND ART

α-lipoic acid (ALA), or thioctic acid, is an endogenous fatty acid with antioxidant properties which acts as a *scavenger* of free radicals which are released at the extracellular level during inflammatory processes. Due to its molecular structure, lipoic acid is able to change from the oxidized form to the reduced form (ALA/DHLA), participating in oxide-reduction reactions or in reactions with different radical species of oxygen [1]. Lipoic acid is present in the leaves of plants containing mitochondria and non-photosynthetic plant tissues such as potato tubers: even broccoli and spinach are particularly rich thereof, but the greatest alimentary source of ALA are red meats and some offal. However, there are problems of bioavailability for the ALA contained in food since it is present in a complexed form with difficult adsorption. For this reason, the dietary intake can benefit from an integration: the dosages may vary according to the individual characteristics, lifestyle, physical activity, exposure to sunlight and diet.

At an intracellular level, lipoic acid takes part in different antioxidative mechanisms such as the regeneration of reduced glutathione (GSH) and of ascorbic acid. At an extracellular level, it acts as *scavenger* of free radicals [2].

Lipoic acid is known to be a useful supplement for the treatment of Alzheimer, multiple sclerosis, diabetic neuropathy and other diseases [7-9].

Preterm birth (PB) is defined as birth before the 37^{th} completed week of gestation. In developed countries, it occurs in 7.5% of all pregnancies, and is associated with high morbidity and neonatal mortality, since 1990 the incidence of PB has increased by about 20% due to the increase of the use of assisted reproduction techniques.

Given the importance of the several clinical and also socio-economic implications, in research, there are many studies aimed at understanding the risk factors and the physiopathological mechanisms involved. The mechanisms underlying preterm birth are manifold: Dudley et al. (J. of reproductive immunology. 1997, 36 (102), 93-109) in fact define such an event as a real syndrome, making specific reference to a complex inflammatory process.

Particularly in the last 20 years, the role of the cervical length and of inflammatory interleukins has been specifically studied.

In physiological pregnancies, the cervical length is considered regular for values ≥35 mm, there are still uncertainties as to which is the cut-off for correctly identifying pregnancies at risk of PB, and currently based on the conclusions of the majority of works available in literature, a cervicometry of ≤ 30 mm is regarded as a threshold.

As described above, in addition to the ultrasound measurement of the cervix, another very thoroughly studied topic in recent years is that of pro-inflammatory cytokines. In fact, over the last 10 years many studies have shown an increase of the proinflammatory cytokine interleukin 6 (IL-6) at the level of cervico-vaginal secretions, of maternal serum and of the amniotic fluid in women with a risk of PB.[3]

In particular, the shortening and dilation processes of the cervix occur as a result of inflammatory processes involving important changes in the extracellular matrix of fibrous connective tissues [4]. For this reason, in case of threatened miscarriage or preterm delivery, the appropriate tocolytic therapies aimed at silencing the excessive uterine contractility should be associated with actions capable of opposing the morphological changes which take place on the fibrous connective tissues of the cervix.

In vitro tests on fragments of amniochorial membranes; after the pick-up, the fragments were placed in incubation with a control medium alone, or TNF alone, with or without a pretreatment with ALA, demonstrated that pre-treatment with ALA is able to inhibit both the weakening of membranes induced by TNF production and the release of MMP9 and cytokine-induced prostaglandin E2 [10-11].

A miscarriage or preterm delivery is a devastating event with social and health implications of great impact. Therefore, there is a clear need to identify new strategies for reducing the number of PB in women at risk.

### SUMMARY OF THE INVENTION

The subject of the present invention is lipoic acid to be administered vaginally for use in the treatment and/or prevention of threatened miscarriage or preterm delivery, said lipoic acid in the form of an enantiomer or mixtures thereof and/or pharmaceutically acceptable organic or inorganic salts thereof.

The subject of the present invention is also a pharmaceutical composition to be administered vaginally, said composition having an acid pH; said composition including lipoic acid, in a dosage from 10 mg to 2 g but also from 0.1 mg to 2 g, and at least one other pharmaceutically acceptable ingredient for use in the treatment and/or prevention of threatened miscarriage or preterm delivery. Vaginal administration has the following advantages compared to the usual oral administration: fast action, reduced dosage, better bioavailability. In fact, since vaginal administration prevents the hepatic metabolic processes (first step effect), it allows effective concentrations of lipoic acid to be achieved in the myometrium layer, in fetal membranes and in the uterine cervix. Systemic administration would not obtain similar results also due to a much more widespread distribution of the molecule in all of the organism sites. A further advantage of vaginal administration is represented by the absence of interaction with other substances (food, drinks) which are known to limit the intestinal absorption processes of lipoic acid, whose preferred supplementation is therefore between meals (C. H. Gleiter, Eur J ClinPharmacol (1996) 50: 513-514). However, often the administration of lipoic acid supplements (commonly characterized by high dosages of such a substance) involves the onset of gastrointestinal disorders due to the acid and irritating nature of the lipoic acid itself. These issues lead patients to take such supplements with meals, thus limiting the pharmacokinetics and efficacy thereof.

In the prior art, it has already been assumed that the (dietary) supplementation with ALA may be useful in the prevention of preterm premature rupture of the fetal membranes. In addition to this assumption, other studies show the ability of ALA to interact with the prostanoid receptors EP2 and EP4 by stimulating the production of cAMP, a potent immunosuppressant. (Salinthone S et al. Journal of Neuroimmunology 2008, 1-2, 46-55). This could have an interesting implication in processes adapted to maintain the uterine quiescence, counteracting the spontaneous preterm uterine contractions safely and free of side effects. The use of lipoic acid, according to the invention, in obstetrics would therefore oppose the common tocolytic agents currently representing the most common therapeutic approach against threatened miscarriage and preterm delivery and which have many limitations in terms of safety (Higby et al. AJOG, 1993, 168 (4), 1247-1259).

### BRIEF DESCRIPTION OF THE DRAWINGS

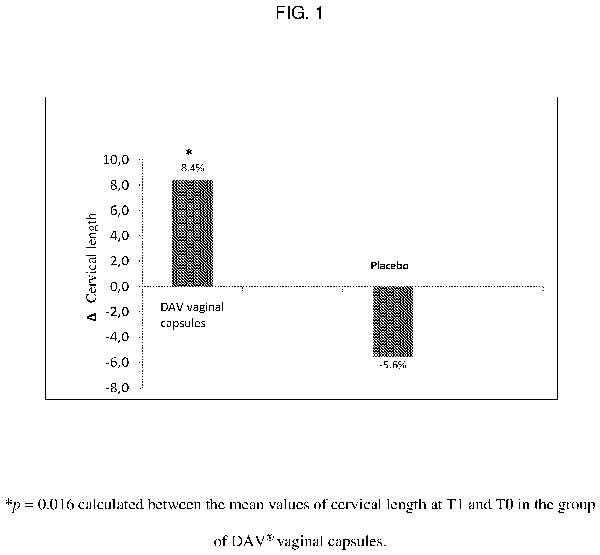

Fig. 1 shows the changes in cervical length between before and after treatment of two groups treated vaginally with ALA and with placebo, respectively.

### DETAILED DESCRIPTION OF THE INVENTION

According to the invention, it is preferred to administer lipoic acid for the treatment of threatened miscarriage or preterm delivery in patients with cervical shortening, that is, characterized by vaginal inflammatory and oxidative conditions due to changes in the vaginal cervix, uterine hypercontractility and/or placental detachment in pregnancy.

It is also preferred to administer lipoic acid to prevent threatened miscarriage or preterm delivery as a result of prenatal diagnostic procedures such as amniocentesis and chorionic villus sampling, assisted reproductive technologies and obstetric surgery.

The composition according to the invention is characterized by an acid pH (preferably 3.5-4.5), in order not to disrupt the physiological conditions of the vaginal environment.

Therefore, the composition of the invention is preferably in the form of vaginal suppositories, rigid or soft vaginal capsules, vaginal cream.

According to the invention, preferably stabilizers are selected from the group consisting of microcrystalline cellulose, sodium carboxymethyl cellulose, medium-chain triglycerides and sodium alginate.

According to the invention, preferably anti-agglomerant agents are selected from the group consisting of magnesium stearate and silicon dioxide.

According to the invention, preferably preservatives are selected from the group consisting of sorbic acid, benzoic acid, lactic acid, magnesium oxide.

According to the invention, preferably rebalancing agents are selected from the group consisting of Lactobacilli, potassium salts (i.e. potassium chloride), sodium hyaluronates.

Preferably, a composition according to the invention to be administered vaginally is in the form of a vaginal capsule or a vaginal ovule and includes lipoic acid in amounts from 10 mg to 800 mg, but also amounts from 0.1 mg to 800 mg, preferably from 5 mg to 200 mg, more preferably from 10 mg to 200 mg; even more preferably 10-50 mg. In trials using vaginal capsules containing 50 mg of lipoic acid, the patients reported bothersome itching and subsequent studies were conducted at lower doses with vaginal capsules containing 10 mg of lipoic acid which allows the itching to be reduced while maintaining the clinical efficacy. Said vaginal capsule preferably comprises an enclosure including gelatin or cellulose derivatives. Said vaginal capsule preferably having a total weight from 300 mg to 3 g.

Preferably, a composition according to the invention is in the form of a vaginal ovule and also includes, in addition to lipoic acid, at least one preservative and at least one rebalancing agent. Preferably, a vaginal ovule according to the invention includes, in addition to lipoic acid, lactic acid, EDTA, polyhexamethylene biguanide hydrochloride and potassium chloride.

Preferably, a composition for use according to the invention, said composition in the form of a vaginal capsule, comprises:
Lipoic acid 5-200 mg; preferably 10-200 mg; more preferably 10-50 mg Magnesium stearate 2-50 mg;
Microcrystalline cellulose 100-600 mg;
Silicon dioxide 3-15 mg;
HPMC (hydroxypropylmethyl cellulose) capsule 80-150 mg.

Preferably, a composition for use according to the invention, said composition in the form of a vaginal ovule, comprises:
Lipoic acid 5-200 mg; preferably 10-200 mg; more preferably 10-50 mg
Lactic acid 0.5-10 mg
EDTA 1-10 mg
Polyhexamethylene biguanide hydrochloride 1-10 mg
Potassium chloride 2-20 mg
Semisynthetic triglycerides a.n. to 2 g

The present invention will be better understood in the light of the following embodiments.

### EXPERIMENTAL PART

### EXAMPLE 1 - FORMULATION in vaginal capsules:

Lipoic acid 50 mg
Magnesium stearate 5 mg
Microcrystalline cellulose 295 mg
Silicon dioxide 5 mg
HPMC (hydroxypropylmethyl cellulose) capsule 95 mg

### EXAMPLE 2 - FORMULATION in vaginal ovules comprising lipoic acid, preservatives, emulsifiers and rebalancing agents.

### Example of formulation:

Lipoic acid 50 mg
Lactic acid 1 mg
EDTA 3 mg
Polyhexamethylene biguanide hydrochloride 3 mg
Potassium chloride 4 mg
Semisynthetic triglycerides a.n. to 2 g

### EXAMPLE 3 - DESIGN OF CLINICAL TRIAL

### TRIAL DESIGN

Randomized pilot trial, vaginal capsules according to example 1 vs. placebo Number of participants: 30 (15 per branch)

### PRIMARY OBJECTIVE

In nulliparous patients with cervical length from 30 mm to 20 mm as measured between 24 and 30 weeks of gestation, the trial has the following objective:
- evaluating whether the administration of ALA by the vaginal route is associated with a stabilization of the cervical shortening and with a reduction of the secretion of pro-inflammatory cytokines IL-6, IL1β and TNFα.

### SECONDARY OBJECTIVES

- Gestation week at delivery
- Mode of delivery
- Apgar score, weight and gender of the child
- Vaginal itching/burning

### INCLUSION CRITERIA

- Primigravid between 24 and 30 weeks of gestation
- Cervicometry from 30 mm to 20 mm
- Single pregnancy
- Intact membranes
- Ultrasound dating of pregnancy

### EXCLUSION CRITERIA

- Rupture of membranes
- Proven fetal or chromosomal malformations
- Loss of blood from the genitals
- Contractions
- Therapy with progesterone

### MATERIALS

The women enrolled were randomized into 2 groups:
- 15 were administered with vaginal capsules containing 50 mg ALA (DAV), 1 per day for 30 days
- 15 were administered with vaginal capsules containing placebo

Two visits were scheduled:
1) Enrolment, where cervicometry and vaginal swab are performed for dosing the pro-inflammatory cytokines
2) At the end of the therapy, where cervicometry and vaginal swab are repeated for dosing the pro-inflammatory cytokines

Preliminary results of the trial showed that vaginal treatment with ALA causes a normalizing effect on the fibrous connective tissues of the cervix. In fact, in patients treated with ALA there was a significant reduction of the cervical shortening processes than the placebo group TAB 1 and Fig.1. This figure is probably due to the antioxidant action of ALA which counteracts the tissue changes caused by reactive oxygen species released during the inflammatory processes.

**Table 1: Effects of vaginal lipoic acid on the cervical length.**

| | **Cervicometry (mm)** | **Cervicometry (mm)** | ***p* values** |
|---|---|---|---|
| | **T0** | **T1** | |
| **DAV** | 24.5 ± 3.0 | 26.6±1.9 | *0.016* |
| **Placebo** | 25.2 ±3.0 | 23.8±2.7 | *NS* |

| | | | |
|---|---|---|---|
| The values of cervicometry in the two groups are referred to as ± SD means, and they were compared with a *Student's t-test.* Differences associated with values of *p* < 0.05 between the two time points were considered significant. | | | |

Figure 1 shows the variations of the cervical length in the two groups before and after treatment

In conclusion, the vaginal ALA turns out to be an effective approach in clinical practice in obstetrics and gynecology in patients at risk for preterm birth.

The clinical observations of this trial showed that patients treated with 50 mg of ALA vaginally reported an annoying itching. For this reason, subsequent studies described hereinafter were conducted at lower dose of ALA.

### EXAMPLE 4 - FORMULATION in vaginal capsules (DAV):

| | Composition of one 400 mg capsule |
|---|---|
| Alpha-lipoic acid (ALA) | 10 mg |
| Microcrystalline cellulose | 286 mg |
| Magnesium stearate | 4 mg |
| Silicon dioxide | 4 mg |
| Hydroxypropyl methylcellulose | 94.08 mg |
| Titanium dioxide | 1.92 mg |

### EXAMPLE 5 - DESIGN OF CLINICAL TRIAL

### TRIAL DESIGN

Randomized trial, vaginal capsules of example 4 vs. progesterone and placebo Number of participants: 66 (27 medical device, 27 progesterone, 22 placebo)

### PRIMARY OBJECTIVE

In primigravid patients between the 7^{th} and 12^{th} week of gestation, the trial had the following objective:
- evaluating whether the administration of 10 mg ALA vaginally is associated with the reabsorption of the subchorionic hematoma.

### SECONDARY OBJECTIVES

Reduction or disappearance of:
- pelvic pain
- vaginal bleeding

### INCLUSION CRITERIA

- Women aged 20-40
- Primigravid between 7^{th} e and 12^{th} weeks of gestation
- Presence of pelvic pain
- With or without moderate vaginal bleeding
- Presence of the subchorionic hematoma detected by ultrasound

### EXCLUSION CRITERIA

- Absence of the fetus
- Absence of heartbeat
- Presence of uterine abnormalities
- Presence of fetal abnormalities
- Presence of multiple pregnancies
- Presence of pre-pregnancy or gestational abnormalities
- Diagnosis of allergic reaction to progesterone
- Presence of ongoing therapies with anticoagulants or antihypertensives
- Presence of karyotypic abnormalities

### MATERIALS

The women enrolled to be treated with ALA or progesterone were randomized into 2 groups:
- 27 were administered with vaginal capsules containing 10 mg ALA 1 per day until resolution of the clinical condition
- 27 were administered with vaginal capsules containing 400 mg progesterone daily.

The women in the control group were those who refuse any medical treatment.

Three visits were scheduled:
1) Enrolment
2) 20 days after the first visit
3) 60 days after the first visit

In each visit, a "blinded" operator carried out the ultrasound examination to evaluate the subchorionic hematoma and the subjective (pelvic pain) and objective (vaginal bleeding) symptoms were recorded.

### Results:

In the group of patients treated with vaginal ALA, the resorption of the subchorionic hematoma showed a statistically significant improvement (*p* ≤ 0.05) compared to that of the progesterone group and the control group. There were no significant differences between these two groups with reference to the absorption of the subchorionic hematoma,
in particular:

| | ALA | Progesterone | Controls |
|---|---|---|---|
| Improvement (%) mean ± st. error | 83.9 ± 3.9 | 49.7 ± 2.7 | 48.6 ± 2.5 |

With reference to the disappearance of pelvic pain, a significant reduction was already observed at the second visit in the group treated with ALA with respect to the other two (*p* ≤ 0.05). In contrast, as regards vaginal bleeding, no significant differences were found between the three groups.

| | Visit upon enrolment | | | 2^{nd} visit | | | 3^{rd} visit | | |
|---|---|---|---|---|---|---|---|---|---|
| Symptoms | ALA | Prog | Contr. | ALA | Prog | Contr. | ALA | Prog | Contr. |
| Pel. pain | 100% | 100% | 100% | 0% | 14% | 12% | - | - | - |
| Vaginal bleeding | 54% | 57% | 65% | 0% | 0% | 12% | - | - | - |

In the ALA group, a significant smaller number of abortions (*p* ≤ 0.05) was observed with respect to those present in the progesterone and control groups.

| Spontaneous abortions | | |
|---|---|---|
| ALA | Progesterone | Controls |
| 3 | 6 | 5 |

### EXAMPLE 6 - DESIGN OF CLINICAL TRIAL

### TRIAL DESIGN

Randomized trial, vaginal capsules of example 4 vs. progesterone and placebo Number of participants: 120 (60 medical device, 60 progesterone)

### PRIMARY OBJECTIVE

In primigravid patients between the 7^{th} and 12^{th} week of gestation, the trial had the following objective:
- evaluating whether the administration of 10mg ALA vaginally is associated with the reduction of the number of spontaneous abortions.

### SECONDARY OBJECTIVES

Reduction or disappearance of symptoms related to the risk of spontaneous abortion after therapy

### INCLUSION CRITERIA

- Women aged 20-40
- Primigravid between 7^{th} e and 12^{th} weeks of gestation
- Threat of spontaneous abortion suggested by the following symptoms:
   Abdominal pain and presence of vaginal bleeding

### EXCLUSION CRITERIA

- Absence of heartbeat
- Presence of uterine abnormalities
- Presence of fetal abnormalities
- Presence of multiple pregnancies
- Presence of pre-pregnancy or gestational abnormalities
- Diagnosis of allergic reaction to progesterone
- Presence of ongoing therapies with anticoagulants or antihypertensives
- Presence of karyotypic abnormalities

### MATERIALS

The women enrolled to be treated with ALA or progesterone were randomized into 2 groups:
- 60 were administered with vaginal capsules containing 10 mg ALA 1 per day until resolution of the clinical condition
- 60 were administered with vaginal capsules containing 400 mg progesterone daily.

Three visits were scheduled:
1) Enrolment
2) 20 days after the first visit
3) 60 days after the first visit

### Results:

In the group treated with ALA, both a smaller number of abortions and persistence of symptoms post treatment were observed, in particular, the results obtained with ALA are statistically significant (p ≤ 0.05) than those obtained with progesterone, in particular:

| | ALA | Progesterone |
|---|---|---|
| Abortion | 15% | 25% |
| Absence of symptoms | 85% | 75% |

The dosage of ALA at 10 mg per vaginal route did not cause itching.

### References

[1] Packer L, Witt EH, Tritschler HJ. (1995).alpha-Lipoic acid as a biological antioxidant. Free radical biology & medicine, 19(2):227-50.
[2] Goraca A, Huk-Kolega H, Piechota A, Kleniewska P, Ciejka E, Skibska B. (2011).Lipoic acid - biological activity and therapeutic potential. Pharmacological reports: PR, 63(4):849-58.
[3] Agarwal A, Gupta S, Sharma RK. (2005).Role of oxidative stress in female reproduction. Reproductive biology and endocrinology: RB&E, 3:28.
[4] Sennstrom MB, Ekman G, Westergren-Thorsson G, Malmstrom A, Bystrom B, Endresen U, et al. (2000).Human cervical ripening, an inflammatory process mediated by cytokines. Molecular human reproduction, 6(4):375-81.
[5] Slattery MM, Morrison JJ. (2002).Preterm delivery. Lancet, 360(9344):1489-97.
[6] Dubicke A, Fransson E, Centini G, Andersson E, Bystrom B, Malmstrom A, et al. (2010).Pro-inflammatory and anti-inflammatory cytokines in human preterm and term cervical ripening. Journal of reproductive immunology, 84(2):176-85.
[7] Salinthone S, Schillace RV, Tsang C, Regan JW, Bourdette DN, Carr DW. (2011).Lipoic acid stimulates cAMP production via G protein-coupled receptor-dependent and -independent mechanisms. The Journal of nutritional biochemistry, 22(7):681-90.
[8] Salinthone S, Schillace RV, Marracci GH, Bourdette DN, Carr DW. (2008).Lipoic acid stimulates cAMP production via the EP2 and EP4 prostanoid receptors and inhibits IFN gamma synthesis and cellular cytotoxicity in NK cells. Journal of neuroimmunology, 199(1-2):46-55.
[9] US5728735
[10] Moore R.M., et al. Placenta, 2010, 31, 886-892.
[11] Moore R.M., et al. Biology of Reproduction 2009, 80, 781-787.

## Claims

1. Lipoic acid for use in the treatment and/or prevention of threatened miscarriage or preterm delivery, said lipoic acid in the form of an enantiomer or mixtures thereof and/or pharmaceutically acceptable organic or inorganic salts thereof; wherein said lipoic acid is administered vaginally.

2. Lipoic acid according to claim 1 for use in the treatment of threatened miscarriage or preterm delivery **characterized by** vaginal inflammatory and oxidative conditions due to changes in the vaginal cervix, uterine hypercontractility and/or placental detachment in pregnancy.

3. Lipoic acid according to claim 1 for use in the prevention of threatened miscarriage or preterm delivery as a result of prenatal diagnostic procedures such as amniocentesis and chorionic villus sampling, assisted reproductive technologies and obstetric surgery.

4. A pharmaceutical composition for use according to any one of claims 1-3, said composition having an acid pH, said composition comprising lipoic acid in a dosage from 0.1 mg to 2 g and at least another pharmaceutically acceptable ingredient.

5. A composition for the use according to claim 4, said composition having a pH from 3.5 to 4.5.

6. A pharmaceutical composition for the use according to any one of claims 4-5, wherein said other pharmaceutically acceptable ingredient is selected from stabilizers, anti-agglomerant agents, emulsifiers, preservatives and rebalancing agents.

7. A pharmaceutical composition for the use according to any one of claims 4-6, said composition in the form of vaginal ovules, rigid or soft vaginal capsules, vaginal cream.

8. A pharmaceutical composition for the use according to claim 7 in the form of a vaginal capsule in which the lipoic acid is present in a dosage from 0.1 mg to 800 mg, preferably from 10 mg to 200 mg.

9. A composition for the use according to claim 7 in the form of a vaginal capsule.

## Patentansprüche

1. Liponsäure zur Verwendung bei der Behandlung und/oder Prävention einer drohenden Fehlgeburt oder Frühgeburt, wobei die Liponsäure als ein Enantiomer oder Mischungen davon und/oder pharmazeutisch verträgliche organische oder anorganische Salze davon formuliert ist, wobei die Liponsäure vaginal verabreicht wird.

2. Liponsäure nach Anspruch 1 zur Verwendung bei der Behandlung einer drohenden Fehlgeburt oder Frühgeburt, **gekennzeichnet durch** vaginale inflammatorische oder oxidative Zustände aufgrund von Veränderungen der vaginalen Cervix, uteriner Hyperkontraktilität und/oder Ablösen der Plazenta während der Schwangerschaft.

3. Liponsäure nach Anspruch 1 zur Verwendung bei der Vorbeugung einer drohenden Fehlgeburt oder Frühgeburt infolge pränataler diagnostischer Prozeduren, wie Amniozentese und Chorionzottenbiopsie, Maßnahmen zur künstlichen Befruchtung und obstetrischem Eingriff.

4. Pharmazeutische Zusammensetzung zur Verwendung nach einem der Ansprüche 1-3, wobei die Zusammensetzung einen sauren pH-Wert aufweist, wobei die Zusammensetzung Liponsäure in einer Dosierung von 0,1 mg bis 2 g und mindestens einen weiteren pharmazeutisch verträglichen Bestandteil umfasst.

5. Zusammensetzung zur Verwendung nach Anspruch 4, wobei die Zusammensetzung einen pH-Wert von 3,5 bis 4,5 aufweist.

6. Pharmazeutische Zusammensetzung zur Verwendung nach einem der Ansprüche 4-5, wobei der weitere pharmazeutisch verträgliche Bestandteil ausgewählt ist aus Stabilisatoren, Rieselhilfen, Emulgatoren, Konservierungsmitteln und Mitteln zur Wiederherstellung des Gleichgewichts.

7. Pharmazeutische Zusammensetzung zur Verwendung nach einem der Ansprüche 4-6, wobei die Zusammensetzung als Vaginalovuli, Hart- oder Weichkapseln zur vaginalen Anwendung, Vaginalcreme formuliert ist.

8. Pharmazeutische Zusammensetzung zur Verwendung nach Anspruch 7 in der Form einer Kapsel zur vaginalen Anwendung, in der die Liponsäure in einer Dosierung von 0,1 mg bis 800 mg, vorzugsweise von 10 mg bis 200 mg, vorliegt.

9. Zusammensetzung zur Verwendung nach Anspruch 7, in der Form einer Kapsel zur vaginalen Anwendung.

## Revendications

1. Acide lipoïque pour une utilisation dans le traitement et/ou la prévention d'une fausse-couche ou d'un accouchement prématuré risquant de se produire, ledit acide lipoïque sous la forme d'un énantiomère ou de mélanges d'énantiomères et/ou de sels organiques ou inorganiques pharmaceutiquement acceptables de celui-ci ; ledit acide lipoïque étant administré par voie vaginale.

2. Acide lipoïque selon la revendication 1 pour une utilisation dans le traitement d'une fausse-couche ou d'un accouchement prématuré risquant de se produire caractérisé(e) par des conditions oxydantes et d'inflammation vaginale dues à des modifications dans le col de l'utérus vaginal, une hypercontractilité de l'utérus et/ou un détachement de placenta pendant la grossesse.

3. Acide lipoïque selon la revendication 1 pour une utilisation dans la prévention d'une fausse-couche ou d'un accouchement prématuré risquant de se produire suite à des procédures de diagnostic prénatal telles qu'une amniocentèse et un échantillonnage de villosités choriales, des technologies de reproduction assistée et une opération obstétrique.

4. Composition pharmaceutique pour une utilisation selon l'une quelconque des revendications 1 à 3, ladite composition ayant un pH acide, ladite composition comprenant de l'acide lipoïque en une dose de 0,1 mg à 2 g et au moins un autre ingrédient pharmaceutiquement acceptable.

5. Composition pour une utilisation selon la revendication 4, ladite composition ayant un pH de 3,5 à 4,5.

6. Composition pharmaceutique pour une utilisation selon l'une quelconque des revendications 4 à 5, dans laquelle ledit autre ingrédient pharmaceutiquement acceptable est sélectionné parmi les agents stabilisants, les agents anti-agglomérants, les émulsifiants, les conservateurs et les agents de rééquilibrage.

7. Composition pharmaceutique pour une utilisation selon l'une quelconque des revendications 4 à 6, ladite composition sous la forme d'ovules vaginaux, de gélules vaginales rigides ou molles, d'une crème vaginale.

8. Composition pharmaceutique pour une utilisation selon la revendication 7 sous la forme d'une gélule vaginale dans laquelle l'acide lipoïque est présent en une dose de 0,1 mg à 800 mg, de préférence de 10 mg à 200 mg.

9. Composition pharmaceutique pour une utilisation selon la revendication 7 sous la forme d'une gélule vaginale.
